# EUROPÄISCHE PATENTSCHRIFT

(11) **EP 3 831 216 B1**
(45) Veröffentlichungstag und Bekanntmachung des Hinweises auf die Patenterteilung: **10.04.2024**
(21) Anmeldenummer: 20020583.9
(22) Anmeldetag: 02.12.2020
(51) Int. Cl.: A23L 13/74, A23L 5/41, C12N 1/20, C12R 1/01, C12R 1/44, A23L 13/40, A23B 4/22, A23L 29/00, A23L 33/105, A23L 33/14, A23L 33/135

(54) **VERFAHREN ZUR UMRÖTUNG VON FLEISCHWAREN**
METHOD FOR REDDENING MEAT PRODUCTS
PROCÉDÉ DE RUBÉFACTION DES PRODUITS À BASE DE VIANDE

(30) Priorität: 02.12.2019 DE 102019132648
(43) Veröffentlichungstag der Anmeldung: 09.06.2021
(73) Patentinhaber: world wide WIBERG GmbH, 5020 Salzburg (AT)
(72) Erfinder: ALPERT, Carl-Alfred, 49080 Osnabrück (DE); NEDDERMANN, Tobias, 32602 Vlotho (DE); HÖFLER, Kristina, 84489 Burghausen (DE)
(74) Vertreter: Schneiders & Behrendt Bochum

(56) Entgegenhaltungen:
- WO-A1-2005/013703
- WO-A1-2019/211428
- DE-A1- 10 059 727
- DE-A1- 19 913 437
- DE-A1-102004 007 814
- DE-A1-102006 050 386
- KR-B1- 101 229 379

## Beschreibung

Die Erfindung betrifft die Verwendung von Arginin zur Umrötung von Fleischwaren.

Bei den herkömmlichen Verfahren zur Umrötung von Fleischerzeugnissen werden diesen Nitritpökelsalz und weitere übliche Zusatzstoffe oder auch nitrathaltige Zutaten zugesetzt. Durch das Zusammenwirken von Kochsalz, Nitrit oder anderen Nitratquellen, Pökelhilfsstoffen, fleischeigenen Enzymen und der Pökelflora bewirkt dieser Zusatz die Reifung, Konservierung und auch die Umrötung des Fleischerzeugnisses, sowie die Stabilisierung der Fleischfarbe, die Entwicklung eines typischen Aromas und nimmt Einfluss auf die Fleischbeschaffenheit. Die Umrötung kann je nach Verfahren bei der Herstellung von Brühwurst einige Stunden in Anspruch nehmen.

Diese Pökelung mit anderen Nitratquellen kann bei Rohwurstwaren zu Produkten mit stabilerer Pökelfarbe und einem volleren Aroma führen. Sie erfordert aber längere Reifungszeiten und ist mikrobiologisch riskanter. Die Reifungszeit kann bei großteiligen Einzelstücken und langzeitgereiften Rohwürsten länger als vier Wochen dauern.

Bei den heute vorwiegend durchgeführten Verfahren der normalen Umrötung bei Brühwurst und Kochpökelwaren, wird nahezu ausschließlich mit Nitritpökelsalz gearbeitet. Den so hergestellten Lebensmittel werden zwischen 80 und 160 ppm Nitrit zugesetzt um die gewünschte Pökelfarbe und das typische Pökelaroma zu erreichen. Bei derartig hergestellten Brühwürsten und Kochpökelwaren muss man mit relativ hohen Rest-Nitrit- und Nitratmengen rechnen. Diese Rest-Nitrit- und Nitratmengen, können ohne weiteres mehr als 40 ppm betragen.

Aus DE 199 13 437 A1, DE 100 59 727 A1, WO 2005/013 703 A1, DE 10 2004 007 814 A1 und DE 10 2006 050 386 A1 ist die Umrötung von Fleischwaren mithilfe von nitratreduzierenden Mikroorganismen und pflanzlichen Substraten, die einen natürlichen Nitratgehalt aufweisen, bekannt. Die WO 2019/211 428 A1 offenbart ein Verfahren zur Umrötung von Fleischprodukten mithilfe von nitratreduzierenden Milchsäurebakterien und/oder Mikrokokken. Dabei können Aminosäuren, Peptide/Proteine und Hefeextrakt zugesetzt werden. KR 101 229 379 B1 beschreibt ein Verfahren zur Fragmentierung von Fleischprodukten mithilfe von nitratreduzierenden Mikroorganismen und einem nitrathaltigen Gemüsepulver.

Aufgabe der Erfindung ist daher die Bereitstellung eines Verfahrens zur Umrötung von Fleischerzeugnissen, das schnell und effizient verläuft und ohne die Verwendung von Nitrat/Nitritpökelsalz oder anderen Nitrit- oder Nitratquellen durchgeführt werden kann. Ein weiterhin verfolgtes Ziel ist es die potenziell karzinogenen Rest-Nitrit- und Nitratmengen auf Mengen deutlich unterhalb der traditionell erreichten Werte zu reduzieren

Diese Aufgabe wird durch Verwendung von Arginin zur Umrötung von Fleischerzeugnissen gemäß Anspruch 1 gelöst.

Erfindungsgemäß wird die Umrötung der Fleischerzeugnisse über Mikroorganismen herbeigeführt, die in der Lage sind, Arginin in NO zu überführen. Das NO reagiert sofort ohne Akkumulation in dem Fleischerzeugnis mit Myoglobin zu Nitrosomyoglobin, das nach Erhitzung oder Trocknung (Eiweißdenaturierung) eine stabile Rotfärbung erzeugt, und verbleibt nicht im Fleisch.

Das NO stammt letztlich aus der Aminosäure Arginin des Hefeextraktes, das aber nicht als alleiniges Substrat dienen kann. Nur mit Arginin kann der Prozess nicht gestartet werden. Vom Stoffwechsel her gesehen kommt das NO aus Arginin und Sauerstoff über eine Stickoxidsynthase unter Abspaltung von Citrullin.

Die Reaktion lässt sich durch Zugabe von Aspartat als freie Säure und Aminosäure enthaltenden Substraten, wie Eiweißpulver oder Sojaproteinisolat, signifikant verstärken.

Allerdings sind Staphylokokken und Kocurien nicht ohne Weiteres in der Lage, Arginin in NO umzuwandeln; es bedarf hierzu eines Arginin enthaltenden Hefeextrakts als Substrat.Zur Auswahl solcher Quellen wurde ein Schnelltest entwickelt, der die Eignung aufzeigt und im Weiteren beschrieben ist.

Die Mikroorganismen werden der Fleischmasse in einer Menge von bis zu 1 × 10¹² Kolonie bildenden Einheiten (KbE) pro kg Fleischmasse zugesetzt, insbesondere in einer Menge von etwa 1 × 10¹¹ KbE. Diese Menge hat sich in der Regel als völlig ausreichend für eine rasche Umrötung der Fleischerzeugnisse erwiesen.

Bei den Substraten handelt es sich um Arginin enthaltende Hefeextrakte, die den oben erwähnten Schnelltest bestehen. Welche Bestandteile dieser Substrate geeignet sind, die Mikroorganismen dazu anzuregen, die Umwandlung von Arginin in NO zu bewirken, ist nicht bekannt, die Auswahl der Substrate durch den Schnelltest hat sich aber als zuverlässig erwiesen.

Geeignete Substrate auf Hefebasis sind Hefeextrakte, wie sie im Handel erhältlich sind. Die Substrate werden in einer Menge von bis zu 20 g, vorzugsweise 1 bis 10 g, insbesondere in einer Menge von 2,5 g bis 6 g pro kg Fleischmasse eingesetzt. Bevorzugt sind Mengen von bis zu 10g, besonderes 2 g bis 6 g und insbesondere 2,5 g bis 3,5 pro kg Fleischmasse.

Die Umrötung der Fleischerzeugnisse erfolgt in der Regel bei einer Temperatur von bis zu 60°C, vorzugsweise bis zu 40°C, über eine Zeit von bis zu 4 h. In der Praxis wird das erfindungsgemäß vorbereitete Fleischerzeugnis nach dem Vermengen der Bestandteile und Kuttern zunächst innerhalb von 2 h auf eine Temperatur von 40°C bis 60°C erwärmt und anschließend auf eine Kerntemperatur von bis zu 80°C gebracht. Die Reifung und Umwandlung des Arginin in NO erfolgt innerhalb dieser Erwärmungsphase auf bis zu 60°C. Damit ist der Reifeprozess in sehr kurzer Zeit abgeschlossen, ebenso für Kochpökel- und Rohpökelwaren.

Als besonders geeignet haben sich eine Reihe von Stämmen gezeigt, die nach den Bestimmungen des Budapester Vertrags bei der DSMZ hinterlegt wurden. Es handelt sich um die Stämme:
Staphylococcus xylosus FASP 2 und FASP 3,
Staphylococcus vitulinus FASP 4,
Staphylococcus equorum FASP 1 und FASP 5,
Kocuria salsicia FASP 6,
Kocuria varians FASP 7 und
Staphylococcus carnosus FASP 8.

Es ist davon auszugehen, dass zahlreiche weitere Stämme der Gattungen Staphylococcus und Kocuria die Fähigkeit haben, unter den hier aufgezeigten Verfahrensbedingungen Arginin aus Hefeextrakten in NO umzuwandeln.

Als Einsatzgebiete der Erfindung kommen insbesondere Brüh- und Kochpökelwaren infrage. Es handelt sich dabei um Brühwurst, Brühdauerwurst, Hot Dogs, Kochschinken und dergleichen. Die Erfindung kann auch für die Herstellung von Rohwürsten herangezogen werden und in anderen Einsatzgebieten, in denen derzeit Nitritpökelsalz oder Ersatzstoffe wie beispielsweise andere Nitratquellen verwandt werden, eingesetzt werden.

Offengelegt wird auch die Verwendung von Mikroorganismen der oben genannten Gattungen bei der Umrötung von Fleischerzeugnissen zusammen mit Arginin enthaltenden Hefeextrakten Packungen zur Umrötung von Fleischerzeugnissen enthalten einen oder mehrere Stämme einer oder mehrerer Spezies der Gattungen Staphylococcus oder Kocuria, die in der Lage sind, Arginin in Gegenwart einesHefeextraktesin Stickstoffmonoxid überführen, in gefriergetrockneter oder anderweitig konservierter Form sowie gesondert davon einen Arginin enthaltenden Hefeextrakt.

### Schnelltest

Die Eignung eines Hefeextraktes kann mit dem nachfolgend beschriebenen Schnelltest ermittelt werden.

Die zu testende Quelle wird in wässrige Lösung gebracht und mit einer kleinen Menge eines Stammes von Staphylococcus equorum, der sich als geeignet für die Umwandlung von Arginin in NO erwiesen hat, beispielsweise FASP 1-8, Phosphatpuffer und Glukose versetzt. Die Lösung wird 2 h bei 30°C inkubiert. Anschließend werden 0,5 ml der Lösung mit 0,5 ml Griess-Reagenz versetzt und diese Lösung bei 540 nm fotometrisch vermessen. Liegt der Wert über 0,35, besser über 0,7, noch besser über 1,0 und insbesondere über 1,5, ist der Zusatzstoff verfahrensgemäß geeignet. Bei einem Wert unter 0,35 ist keine ausreichende Funktion gegeben.

### Beispiel

50 kg Schweinefleisch II, 3 mm, 15 kg Schweinebacken, 3 mm, 10 kg Speck, 3 mm und 25 kg Eis werden miteinander gewolft mit 18 g/kg Kochsalz, 3g/kg Phosphat und Ascorbinsäure versetzt, gekuttert und anschließend mit 0,1 g entsprechend 1× 10¹¹ KbE Staphylococcus equorum FASP 5 und 3g/kg Hefeextrakt versetzt. Das Brät wird in Sterildärme mit Kaliber 90 gefüllt und in die Kochkammer gefahren. Dort wird das Brät 2 h bei 40°C Kammertemperatur umröten gelassen und dann bei 78°C Kammertemperatur bis zur Erreichung einer Kerntemperatur von 72°C erhitzt. Die Kerntemperatur wurde nach 2:20 h erreicht. Anschließend werden die Würste kalt abgeduscht und abkühlen gelassen.

### Vergleichsbeispiel

Das Beispiel wurde unter Verwendung von 18 g/kg Nitritpökelsalz wiederholt, wobei auf den Zusatz von Staphylococcus equorum und Hefeextrakt verzichtet wurde. Reifung und Brühen erfolgt nach Standardbedingungen.

Die nach dem Beispiel und Vergleichsbeispiel erhaltenen Produkte stimmten farblich und geschmacklich in allen wesentlichen Parametern überein. Das erfindungsgemäße Verfahren erwies sich damit dem herkömmlichen Verfahren als gleichwertig.

## Patentansprüche

1. Verwendung von Arginin zur Umrötung von Fleischerzeugnissen unter Verwendung von Mikroorganismen, bei dem Fleischmasse mit bis zu 1x 10¹² Kolonie bildenden Einheiten pro kg Fleischmasse einer oder mehreren lebensmitteltauglichen Spezies der Gattungen Staphylococcus oder Kocuria, die in der Lage ist, Arginin in Stickstoffmonoxid zu überführen, und mit bis zu 20 g pro kg Fleischmasse eines Arginin enthaltenden Hefeextraktes versetzt und einem Umröteprozess unterzogen wird wobei das aus dem Arginin freigesetzte Stickstoffmonoxid mit dem Myoglobin des Fleischerzeugnisses zu Nitrosomyoglobin reagiert.

2. Verwendung nach einem der vorstehenden Ansprüche, **dadurch gekennzeichnet, dass** die Fleischmasse mit 1 bis 10 g Hefeextrakt pro kg Fleischmasse versetzt wird.

3. Verwendung nach einem der vorstehenden Ansprüche, **dadurch gekennzeichnet, dass** die Fleischmasse bei einer Temperatur ≤ 60° C Umröten gelassen wird.

4. Verwendung nach einem der vorstehenden Ansprüche **dadurch gekennzeichnet, dass** einer oder mehrere der folgenden Mikroorganismen eingesetzt wird/werden:
Staphylococcus xylosus FASP 2 und FASP 3
Staphylococcus vitulinus FASP 4
Staphylococcus equorum FASP 1 und FASP 5
Kocuria salsicia FASP 6
Kocuria varians FASP 7,
Staphylococcus carnosus FASP 8.

5. Verwendung nach einem der vorstehenden Ansprüche, **dadurch gekennzeichnet, dass** es zur Herstellung von Brühwurst, Brühdauerwurst, Kochwurst und Kochpökelwaren eingesetzt wird.

6. Verwendung nach einem der Ansprüche 1 bis 4, **dadurch gekennzeichnet, dass** es zur Herstellung von Rohwurst und Rohpökelwaren eingesetzt wird.

## Claims

1. Use of Arginine for reddening meat products using microorganisms, wherein up to 1 × 10¹² colony-forming units per kilogram meat mass of one or more food-stable species of the Staphylococcus or Kocuria type, which are capable of converting arginine into nitrogen, and up to 20 g per kg meat mass of an arginine-containing yeast extract are added to meat mass and the meat mass is subjected to a reddening process, wherein the nitrogen monoxide released from the arginine reacts with the myoglobin of the meat product to produce nitrosomyglobin.

2. Use according to the preceding claim, **characterized in that** 1 to 10 g of yeast extract pro kg meat mass is added to the meat mass.

3. Use according to one of the preceding claims, **characterized in that** reddening of the meat mass takes place at a temperature of ≤ 60°C.

4. Use according to one of the preceding claims, **characterized in that** one or more of the following microorganisms is/are used:
Staphylococcus xylosus FASP 2 and FASP 3
Staphylococcus vitulinus FASP 4
Staphylococcus equorum FASP 1 and FASP 5
Kocuria salsicia FASP 6
Kocuria varians FASP 7,
Staphylococcus carnosus FASP 8.

5. Use according to any one of the preceding claims, **characterised in that** it is used for the production of boiled sausages, summer sausages, cooked sausages and cooked cured food products.

6. Use according to any one of claims 1 to 4, **characterised in that** it is used for the production of raw sausages and raw cured food products.

## Revendications

1. Utilisation d'arginine pour rougir des produits de viande en utilisant des micro-organismes, dans laquelle la masse de viande est mélangée avec jusqu'à 1 × 10¹² unités formant colonies par kg de masse de viande d'une ou plusieurs espèces de qualité alimentaire du genre Staphylococcus ou Kocuria, capables de transformer l'arginine en monoxyde d'azote, et avec jusqu'à 20 g par kg de viande d'un extrait de levure contenant de l'arginine et soumise à un processus de rougissement, le monoxyde d'azote libéré par l'arginine réagissant avec la myoglobine du produit de viande pour former de la nitrosomyoglobine.

2. Utilisation selon l'une quelconque des revendications précédentes,
**caractérisée en ce que** la masse de viande est mélangée avec 1 à 10 g d'extrait de levure par kg de masse de viande.

3. Utilisation selon l'une quelconque des revendications précédentes,
**caractérisée en ce que** la masse de viande est laissée à rougir à une température ≤ 60 °C.

4. Utilisation selon l'une quelconque des revendications précédentes,
**caractérisée en ce qu'**un ou plusieurs des micro-organismes suivants sont utilisés :
Staphylococcus xylosus FASP 2 et FASP 3,
Staphylococcus vitulinus FASP 4,
Staphylococcus equorum FASP 1 et FASP 5,
Kocuria salsicia FASP 6,
Kocuria varians FASP 7,
Staphylococcus carnosus FASP 8.

5. Utilisation selon l'une quelconque des revendications précédentes,
**caractérisée en ce qu'**elle est utilisée pour la fabrication de saucisses à cuire, de saucissons à cuire, de saucisses cuites et de produits de salaison cuits.

6. Utilisation selon l'une quelconque des revendications 1 à 4, **caractérisée en ce qu'**elle est utilisée pour la fabrication de saucisses crues et de produits de salaison crus.
